# EUROPÄISCHE PATENTSCHRIFT

(11) **EP 4 003 202 B1**
(45) Veröffentlichungstag und Bekanntmachung des Hinweises auf die Patenterteilung: **07.01.2026**
(21) Anmeldenummer: 20740638.0
(22) Anmeldetag: 21.07.2020
(51) Int. Cl.: A61B 18/04, A61B 18/00

(54) **ELEKTRODENANORDNUNG**
ELECTRODE ASSEMBLY
ENSEMBLE D'ÉLECTRODE

(30) Priorität: 23.07.2019 EP 19187863
(43) Veröffentlichungstag der Anmeldung: 01.06.2022
(73) Patentinhaber: Erbe Elektromedizin GmbH, 72072 Tübingen (DE)
(72) Erfinder: BRANDT, Tjark, 72072 Tübingen (DE); SCHNITZLER, Uwe, 72074 Tübingen (DE); HEYM, Johannes, 72070 Tübingen (DE); WALZ, Martin, 72076 Tübingen (DE)
(74) Vertreter: Rüger Abel Patentanwälte PartGmbB
(86) Internationale Anmeldenummer: PCT/EP2020/070580
(87) Internationale Veröffentlichungsnummer: WO 2021/013847

(56) Entgegenhaltungen:
- WO-A1-2019/082765
- DE-A1- 10 030 111
- DE-A1- 102017 127 976
- US-A- 5 207 675
- US-A1- 2008 039 834

## Beschreibung

Die Erfindung betrifft eine Elektrodenanordnung für ein elektrochirurgisches Instrument, insbesondere für ein Instrument zur Plasmakoagulation von biologischem Gewebe.

Instrumente zur Gewebekoagulation sind aus verschiedenen Druckschriften so wie aus der Praxis bekannt. Es wird dazu auf die DE 10 2011 116 678 A1 sowie auf die DE 699 28 370 T2 verwiesen. Beide Druckschriften offenbaren Instrumente mit Elektroden, die zum Beispiel ringförmig ausgebildet sind und aus einem geeigneten Material, wie u.a. auch Wolfram, bestehen können, das sich durch seine Hitzebeständigkeit auszeichnet.

Weiter ist aus der DE 100 30 111 A1 ein Plasmakoagulationsinstrument mit einem schlauchartigen Instrumentenkörper bekannt, in dessen Lumen eine sechseckige, aus einem Metall bestehende Elektrode angeordnet ist. Diese ist mit einer elektrischen Zuleitung verbunden, um die an dem distalen Ende des Schlauchs angeordnete Elektrode mit HF-Spannung versorgen zu können. Von der am distalen Ende der plättchenförmigen Elektrode ausgebildeten Spitze geht eine elektrische Entladung aus, wodurch eine Plasmastrom, insbesondere ein Edelgas-Plasmastrom erzeugt werden kann. Der die Elektrode umspülende Gasstrom dient zugleich der Abführung von Wärme aus der Elektrode, wodurch deren übermäßige Erhitzung vermieden werden soll. Durch die Wärmeabfuhr soll außerdem eine Minimierung des Abbrandverhaltens am Entladungsabschnitt der Elektrode erreicht und dadurch die Lebensdauer des Instruments erhöht werden. DE 10 2017 127 976 A1 offenbart zudem, dass die Elektrode eine Plasmainstruments aus Wolfram bestehen kann, weil es beständiger ist, als Edelstahl.

Eine effiziente Kühlung des Elektrodenplättchens durch den Gasstrom erfordert aber einen hohen Gasfluss, was nicht immer gewünscht ist.

WO 2019/082765 A1 beschreibt eine Elektrode für ein elektrochirurgisches Instrument. mit einem Grundkörper aus einem Metall oder einer Metalllegierung, z.B. Edelstahl. Der Grundkörper ist mit einem Überzug beschichtet aufweisend eine Zwischenschicht mit einer relativ zum Grundkörper höheren thermischen Leitfähigkeit. Eine Oberflächenschicht mit einer hohen thermischen Leitfähigkeit ist auf der Zwischenschicht angeordnet und hat in eine nicht-metallischen Matrix eingebettete Metallpartikel. Ferner beschreibt US 2008/039834 A1 ein Plasmainstrument mit einer aus flexiblem Draht ausgeführten Elektrode aus Tungsten. Die Elektrode weist eine Beschichtung aus Silber auf.

Aus der WO 2005/046495 A1 ist es darüber hinaus bekannt, eine Zündelektrode aus einem Wolframdraht zu verwenden, dessen Ende am distalen Ende eines ansonsten schlauch- oder rohrförmigen Instrumentenkörpers platziert ist. Der in dem Lumen dieses Körpers angeordnete Wolframdraht ist in einem gewissen Abstand zu seinem distalen Ende von einem Plättchen gehalten, an dem er befestigt ist und das zu seiner Kühlung dient. Allerdings ist die Wärmeabfuhr von dem Wolframdraht durch die Übergangsstelle zwischen dem Plättchen und dem Wolframdraht behindert.

Durch Abschmelzen der Elektroden können Partikel, insbesondere Metallpartikel, in den Funken und/oder den Plasmastrom und somit letztlich in lebendes Gewebe geraten, was zunehmend abgelehnt wird.

Es ist deswegen Aufgabe der Erfindung, ein Konzept anzugeben, mit dem sich die Materialabgabe eines elektrochirurgischen, insbesondere plasmachirurgischen Instruments im Einsatz verringern lässt.

Diese Aufgabe wird mit der Elektrodenanordnung nach Anspruch 1 und bei einem Instrument nach Anspruch 13 gelöst:
Die erfindungsgemäße Elektrodenanordnung weist eine Elektrode auf, an der eine distal orientierte Spitze ausgebildet ist. Der Elektrodenquerschnitt nimmt in proximaler Richtung von der Spitze weg kontinuierlich oder in mindestens einer Stufe zu. Die Elektrode besteht aus einer Materialkombination, deren Wärmeleitfähigkeit vorzugsweise größer als 20W/(m*K) ist, womit sich damit ein sehr stark reduzierter Elektrodenabbrand erreichen lässt. Die Elektrode ist mit einem wärmeleitenden Überzug versehen, wodurch die Lebensdauer der Elektrode und auch die Lebensdauer eines mit der Elektrode ausgerüsteten Instruments erhöht ist. Unabhängig von der Wärmeleitfähigkeit der Elektrode bewirkt der wärmeleitende Überzug eine Erhöhung der Lebensdauer der Elektrode gegenüber einer gleichen Elektrode ohne Überzug. Der Überzug erstreckt sich in distaler Richtung vorzugsweise bis in die Nähe des distalen Endes der Elektrode oder überdeckt das distale Ende derselben.

Der Elektrodenquerschnitt kann sich ausgehend von der distalen Spitze stufenartig oder auch kontinuierlich erhöhen, bis die Elektrode die Wandung des Lumens berührt, in dem sie angeordnet ist. Findet die Querschnittsvergrößerung von der Spitze zu einem proximalen Teil der Elektrode stufenweise statt, können dazu ein oder mehrere Stufen vorgesehen sein. Die Spitze der Elektrode (und der an die Spitze angrenzende Flankenbereich der Elektrode) ist diejenige Stelle, von der typischerweise ein Funken oder ein Plasmastrom ausgeht. Die Spitze und der sich unmittelbar daran anschließende Teil der Elektrode bilden somit denjenigen Bereich, in dem sich der Entladungsfußpunkt der elektrischen Entladung befindet. An diesem Fußpunkt findet eine Stromkonzentration statt, die zugleich eine Wärmequelle bildet. An dem Entladungsfußpunkt kann das Grundmaterial der Elektrode frei liegen, d.h. der Überzug nicht vorhanden oder während des Betriebs entfernt worden sein. Durch beide Maßnahmen, nämlich die Querschnittszunahme der Elektrode in proximaler Richtung und durch Nutzung einer Materialkombination für die Elektrode, deren Wärmeleitfähigkeit vorzugsweise größer als 20W/(m*K) ist, wird an dem Elektrodenfußpunkt entstehende Wärme weit wirksamer dissipiert als es bislang bei Verwendung von Elektroden der gleichen Bauform aus rostfreiem Stahl oder Chrom-Nickel-Stahl der Fall war. Die erfindungsgemäße Elektrode zeichnet sich somit insbesondere dadurch aus, dass deren Wärmeleitfähigkeit von der Spitze, gemessen in proximaler Richtung und/oder gemessen quer zur proximalen Richtung, größer als die Wärmeleitfähigkeit von Edelstahl ist.

Vorzugsweise weist die Elektrode einen von ihrer Spitze ausgehenden, sich in proximaler Richtung um wenigstens 2,5 mm erstreckenden Abschnitt auf, dessen Wärmekapazität kleiner als 4,17 mJ/K ist. Dies trägt zur schnellen lokalen kleinräumigen (z.B. auf wenige Quadratmillimeter begrenzte) Erwärmung der Elektrodenspitze und zur Fixierung des Entladungsfußpunkts in diesem Bereich bei. Die Beschichtung kann so bereichsweise, z.B. in kleinen der distalen Spitze nahen Bereichen schmelzen.

Die Kombination einer Elektrodengeometrie, bei der Elektrodenquerschnitt von der Spitze in proximaler Richtung weg zunimmt, wobei die Elektrode aus einer hochwärmeleitfähigen Materialkombination ausgebildet ist, ermöglich die Nutzung einer Spitze mit besonders geringem Rundungsradius, der insbesondere kleiner sein kann als 1/10 der maximalen Querabmessung der Elektrode. Dadurch wird an der Elektrodenspitze eine hohe Feldstärke erreicht, die auch bei niedrigen HF-Spannungen und -Strömen zur Ausbildung eines Funkens und eines Plasmas führen kann. Die Elektrode ist dann besonders zündwillig.

Die Elektrode ist plättchenförmig ausgebildet, wobei die Erhöhung des Elektrodenquerschnitts durch eine entlang der Axialrichtung in proximaler Richtung von der Spitze weg zunehmende Querabmessung der Elektrode erreicht wird. Diese Querabmessung kann kontinuierlich zunehmen, wodurch eine besonders gute Wärmeableitung erreicht wird. Die kontinuierliche Querschnitts- und Querabmessungszunahme kann erreicht werden, indem sich an die Spitze der Elektrode stufenlos ausgebildete Kanten anschließen.

Die Elektrode ist als Plättchen ausgebildet. Das Plättchen kann zwei Flachseiten aufweisen, die durch Schmalseiten miteinander verbunden sind. Zwei aufeinander zu laufende Kanten können sich an der Spitze treffen, die das distale Ende der Elektrode bildet. Zwischen den Schmalseiten und den Flachseiten können Kanten ausgebildet sein. Eine solche Elektrode kann beispielsweise als Blechzuschnitt bereitgestellt werden.

Im Folgenden ist ein Beispiel der Ausgestaltung der Elektrode als Draht offenbart, dieses Beispiel ist jedoch nicht beansprucht:
Die Elektrode kann auch als Drahtelektrode, somit als dünner Stab ausgebildet sein, dessen distales Ende die Spitze bildet. Der Draht (Nadel, dünner Stab) kann mit einem Halteteil verbunden sein, das sich diametral durch ein Lumen einer Sonde erstreckt und Teil der Elektrode ist.

Gemäß einer der Erfindung besteht die Elektrode aus einer Materialkombination, die dadurch gebildet ist, dass die Elektrode aus einem Grundkörper besteht, der mindestens eine Fläche aufweist, an der eine Wärmeableiteinrichtung angebracht ist. Die Wärmeableiteinrichtung erstreckt sich dabei in distaler Richtung vorzugsweise bis zu der Spitze der Elektrode zumindest bis an einen Bereich, der im Betrieb von dem Entladungsfußpunkt eingenommen wird. Die dort entstehende Wärme kann somit unmittelbar auf die Wärmeableiteinrichtung übertragen werden, ohne dass eine Wärmeübertragung von der Elektrode auf die Wärmeableiteinrichtung nötig wäre. Die Wärmeableiteinrichtung steht mit anderen Worten in direktem Kontakt mit der Wärmequelle, hier in Gestalt des Entladungsfußpunkts. In proximaler Richtung erstreckt sich die Wärmeableiteinrichtung vorzugsweise mindestens bis in einen Bereich der Elektrode, in dem diese ihre maximale Querabmessung aufweist.

Im einfachsten Fall besteht dazu die Elektrode aus einem Grundmaterial, auf das als Wärmeableiteinrichtung ein wärmeleitender Überzug, zum Beispiel in Form einer wärmeleitenden Beschichtung, aufgebracht ist. Diese Schicht erstreckt sich vorzugsweise bis zu der Spitze der Elektrode und über große Teile der Flachseiten oder über die gesamten Flachseiten der Elektrode. Die Beschichtung kann sich auch über die Schmalseiten der Elektrode erstrecken. Ist die Elektrode beispielsweise aus Edelstahl oder einem anderen weniger gut wärmeleitenden Material ausgebildet, ist die Wärmeableiteinrichtung aus einem besonders gut wärmeleitenden Material, wie beispielsweise Silber, kohlenstoffähnlichem Diamant (DLC) oder dergleichen ausgebildet. Vorzugsweise aber ist die Wärmeableiteinrichtung aus einem metallischen Material ausgebildet, das auch stromleitend ist, sodass die Wärmeableiteinrichtung zum Beispiel in Gestalt der wärmeableitenden Schicht zur Stromleitung beiträgt und in direktem Kontakt mit dem Entladungsfußpunkt kommen kann. Die Wärmeableiteinrichtung kann auch aus einem gut wärmeleitenden keramischen Material, z.B. AlN (Aluminiumnitrid) bestehen. Das keramische Material kann elektrisch leitend oder elektrisch isolierend ausgebildet sein. Vorzugsweise ist die Wärmeableiteinrichtung, die z.B. als wärmeleitende Beschichtung ausgebildet ist, auch besonders gut stromleitend. Insbesondere ist es vorteilhaft, wenn die elektrische Leitfähigkeit der Beschichtung größer ist als die elektrische Leitfähigkeit des Grundmaterials. Bevorzugter Weise ist die Schicht wenigstens teilweise aus Silber. Es kann sich um Reinsilber oder eine Silberlegierung oder um einen mehrschichtigen Aufbau handeln, bei dem wenigstens eine Schicht, vorzugsweise die an der Oberfläche liegende Schicht aus Silber oder einer Silberlegierung besteht.

Der Schichtaufbau kann eine haftungsbeeinflussende Schicht umfassen, z.B. eine zwischen der oberflächennahen Schicht und dem Grundmaterial angeordnete Zwischenschicht. Die Zwischenschicht kann insbesondere eine Schicht sein, die die Haftung der oberflächennahen Schicht auf dem Grundmaterial beeinflusst. Insbesondere kann die Zwischenschicht eine Haftschicht sein, die die Beschichtung des Grundmaterials (Rostfreier Stahl) mit dem Beschichtungsmaterial (Silber) begünstigt. Die Haftschicht kann in Betrieb die Umverteilung des Beschichtungsmaterials von der distalen Spitze weg fördern.

Vorzugsweise ist die Schmelztemperatur des Oberflächenmaterials niedriger als die Schmelztemperatur des Grundmaterials. Ist eine Zwischenschicht vorhanden, ist die Schmelztemperatur der Zwischenschicht niedriger als die Schmelztemperatur des Grundmaterials und höher als oder genauso hoch wie die Schmelztemperatur des Materials der Oberflächenschicht. Die Schmelztemperatur der Zwischenschicht kann aber auch kleiner als die Schmelztemperatur des Beschichtungsmaterials sein.

Weiter vorzugsweise ist die Wärmeleitfähigkeit des Materials der Oberflächenschicht höher als die Wärmeleitfähigkeit des Grundmaterials. Ist eine Zwischenschicht vorhanden, ist die Wärmeleitfähigkeit des Materials der Zwischenschicht vorzugsweise höher als die Wärmeleitfähigkeit des Grundmaterials und niedriger als, höher als, oder gleich wie die Wärmeleitfähigkeit des Materials der Oberflächenschicht.

Bei einer besonders bevorzugten Ausführungsform weist die Wärmeableiteinrichtung somit eine elektrische Leitfähigkeit und insbesondere auch eine thermische Leitfähigkeit auf, die jeweils größer sind als die elektrische und thermische Leitfähigkeit des Materials des Grundkörpers.

Als Grundmaterial eignen sich insbesondere Legierungen, die Eisen und/oder Chrom und/oder Nickel enthalten. Außerdem können als weitere Legierungsbestandteile Kohlenstoff und/oder Mangan und/oder Phosphor und/oder Schwefel und/oder Silizium und/oder Nickel und/oder Stickstoff und/oder Molybdän vorhanden sein. Ein als Grundmaterial bevorzugter Edelstahl hat folgende Zusammensetzung:

| | Fe | C | Cr | Mn | P | S | Si | Ni | N | Mo |
|---|---|---|---|---|---|---|---|---|---|---|
| min | | 0,05 | 16, 0 | | | | | 6, 0 | | |
| max | 47, 605 | 0,15 | 19, 0 | 2, 0 | 0,045 | 0,15 | 2, 0 | 9, 5 | 0, 11 | 0, 8 |

Als Material für die Zwischenschicht eignen sich insbesondere Gold oder Nickel oder deren Legierungen.

In der Zeichnung sind Ausführungsbeispiele der Erfindung veranschaulicht. Es zeigen:
Figur 1 ein erfindungsgemäßes Instrument, das zugehörige speisende Gerät und eine Neutralelektrode, in sehr schematisierter, teilweise perspektivischer Darstellung,
Figur 2 das distale Ende des Instruments nach Figur 1, in perspektivischer schematisierter Längsschnittdarstellung,
Figur 3 die Elektrode des Instruments nach Figur 2, in Seitenansicht,
Figur 4, 5 und 6 verschiedene Querschnitte der Elektrode nach Figur 3,
Figur 7 das Instrument nach den Figuren 2 bis 6, in geschnittener perspektivischer Darstellung während des Betriebs,
Figur 8 eine abgewandelte Ausführungsform einer Elektrode für ein Instrument gemäß Figur 2,
Figur 9 und 10 eine weitere Ausführungsform einer Elektrode für ein Instrument gemäß Figur 2.

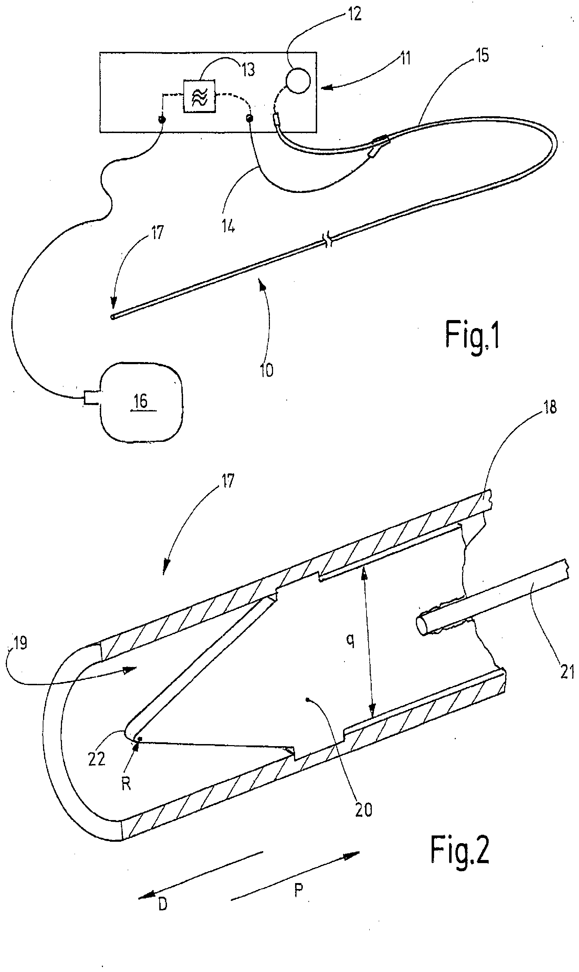

In Figur 1 ist ein Instrument 10 veranschaulicht, das zur plasmagestützten Gewebebehandlung dient. Die Gewebebehandlung kann die Ablation, Koagulation, das Schneiden oder anderweitige Behandlungsarten umfassen.

Das Instrument 10 ist an ein Gerät 11 angeschlossen, das eine Gasquelle 12, zum Beispiel eine Argonquelle, sowie einen Generator 13 zur elektrischen Speisung des Instruments 10 enthält. Diese ist über entsprechende Anschlussmittel mit einer Leitung 14 verbunden, die zu dem Instrument 10 führt und die in eine Leitung 15 eingeführt ist, über die das Instrument 10 mit Gas gespeist wird. Der Generator 13 ist außerdem über entsprechende Verbindungsmittel mit einer Neutralelektrode 16 verbunden, die vor Benutzung des Instruments 10 an dem Patienten anzubringen ist. Die nachstehende Beschreibung gilt aber auch für Instrumente mit anderer Neutralelektrodenkonfiguration.

Das Instrument 10 weist ein distales Ende 17 auf, das gesondert in Figur 2 veranschaulicht ist. Wie ersichtlich gehört zu dem Instrument 10 ein Rohr oder Schlauch 18, das bzw. der ein Lumen 19 umgibt, das am distalen Ende 17 des Schlauchs 18 offen ist. Im Bereich des distalen Endes 17 kann der Schlauch 18 mit einer inneren oder äußeren Verstärkung ersehen sein, beispielsweise in Gestalt einer Keramikhülse, was in Figur 2 nicht näher dargestellt ist. Der Schlauch 18 kann somit ein- oder mehrschichtig aufgebaut sein. Beispiele für Instrumente mit in das offene Ende des Schlauchs 18 eingesetzter Keramikhülse sind der WO 2005/046495 A1 zu entnehmen.

In dem Lumen 19 ist eine Elektrode 20 angeordnet, die mit einem zu der Leitung 14 gehörenden Draht 21 elektrisch verbunden ist, der sich durch das Lumen 19 erstreckt. Der Draht 21 kann mit der Elektrode 20 verschweißt oder auch mechanisch, beispielsweise durch Krimpen, verbunden sein.

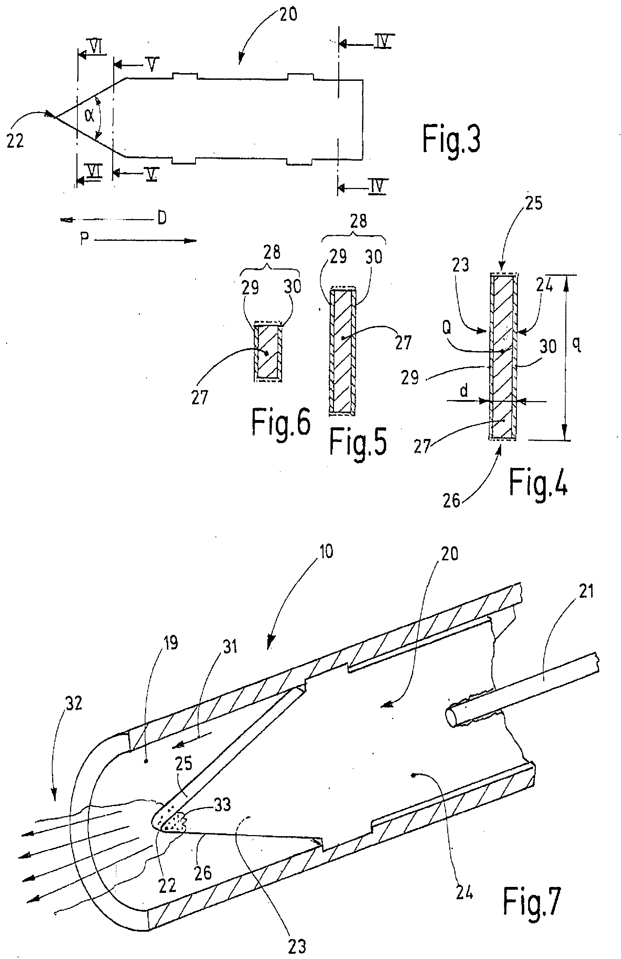

Die Elektrode 20 weist vorzugsweise die in Figur 3 veranschaulichte Grundform auf. An ihrem distalen Ende ist an der Elektrode 20 eine scharfe oder allenfalls leicht gerundete Spitze 22 ausgebildet, deren Rundungsradius R (Figur 2) möglichst gering und vorzugsweise geringer als ein Zehntel der Querabmessung q ist, die quer zur Axialrichtung zu messen ist und mit dem Innendurchmesser des Lumens 19 weitgehend übereinstimmt. Die Elektrode 20 ist vorzugsweise plattenförmig ausgebildet, d.h., ihre Dicke ist wesentlich geringer als ihre Querabmessung q. Dies ergibt sich zum Beispiel aus Figur 4, die den Querschnitt der Elektrode 20 an der in Figur 3 strichpunktierten Schnittlinie IV-IV zeigt. Die Dicke d ist geringer als 1/5 vorzugsweise geringer als 1/10 der Querabmessung q.

Wie weiter aus Figur 4 ersichtlich, weist die Elektrode 20 zwei Flachseiten 23, 24, die durch Schmalseiten 25, 26 verbunden sind. Es ergibt sich somit ein insgesamt viereckiger, vorzugsweise rechteckiger Querschnitt Q, der von den Flachseiten 23, 24 und den Schmalseiten 25, 26 umgrenzt ist. Der viereckige Querschnitt kann auch ein oder mehrfach, z.B. s-förmig gebogen sein.

Die Elektrode 20 weist an ihrem distalen Ende einen Verjüngungsbereich auf, in dem die ansonsten parallel zueinander verlaufenden Schmalseiten 25, 26 zu der Spitze 22 hin konvergent angeordnet sind. Die konvergent aufeinander zu verlaufenden Abschnitte der Schmalseiten 25, 26 können, wie Figur 3 zeigt, gerade oder auch konvex oder konkav ausgebildet sein. Sie legen zwischen einander einen Winkel α fest, der vorzugsweise im Bereich von 20° und 100° liegt.

Wie die Querschnitte V-V und VI-VI zeigen, die gesondert in den Figuren 5 und 6 dargestellt sind, nimmt der Querschnitt der Elektrode 20 zur Spitze 22 hin in distaler Richtung D ab oder mit anderen Worten in proximaler Richtung P zu. Dabei kann die Dicke d der Elektrode 20 im Verjüngungsbereich zur Spitze 22 hin wie die Figuren 5 und 6 zeigen konstant bleiben. Die Dicke d kann jedoch zu der Spitze 22 hin auch abnehmen. Jedenfalls nimmt aber die Querabmessung Q in dem Verjüngungsbereich zur Spitze 22 hin ab.

Bei einer bevorzugten Ausführungsform der Erfindung weist die Elektrode 20 einen Mehrschichtaufbau auf, wie er aus den Figuren 4, 5 und 6 hervorgeht. Die Elektrode 20 weist dazu einen Elektrodengrundkörper 27 auf, der zumindest an seinen Flachseiten 23, 24 jedoch optional auch an seinen Schmalseiten 25, 26 mit einer Wärmeableiteinrichtung 28 verbunden ist. Diese besteht im vorliegenden Ausführungsbeispiel aus einer vollflächigen Beschichtung der Flachseiten des Elektrodengrundkörpers 27 mit thermisch leitenden Überzügen 29, 30. Im Ausführungsbeispiel kann der Grundkörper 27 aus Edelstahl bestehen, während die Überzüge 29, 30 aus einem anderen Material mit besserer Wärmeleitfähigkeit und/oder besserer elektrischer Leitfähigkeit bestehen. Als besonders geeignet hat sich dafür Silber erwiesen. Mögliche andere Überzüge besehen aus Aluminium und/oder Kupfer und/oder Hartmetall und/oder DLC und/oder Wolfram und oder einer Schicht, z.B. Metallschicht mit darin eingebettetem CBN (kubisches Bornitrid), Diamantpulver oder ähnlich gut wärmeleitendem Material.

Das insoweit beschriebene Instrument arbeitet wie folgt:
Wie in Figur 7 veranschaulicht, wird das Lumen 19 des Instruments 10 in Betrieb von einem Gasstrom 31 durchflossen, der von der Gasquelle 12 herrührt. Dieser Gasstrom (vorzugsweise Argonstrom) streicht entlang beider Flachseiten 23, 24 der Elektrode 20. Über den Draht 21 wird die Elektrode 20 zugleich mit hochfrequentem elektrischen Strom gespeist. Die Arbeitsfrequenz des Generators 13 und somit die Frequenz des Stroms liegt dabei vorzugsweise oberhalb von 100 kHz, vorzugsweise oberhalb von 300 kHz, weiter vorzugsweise oberhalb von 500 kHz. An der Spitze 22 und einem angrenzendem Bereich derselben verlässt der Strom die Elektrode 20 und bildet einen zu dem nicht weiter veranschaulichten biologischen Gewebe des Patienten überspringenden Funken oder ein dorthin fließendes Plasma 32. Der Fußpunkt 33 des Funkens oder Plasmas berührt dabei die Schmalseiten 25, 26, insbesondere aber die Flachseiten 23, 24 der Elektrode 20, wobei dieser Fußpunktbereich 33 z.B. weniger als 1/10 der axialen (in Proximalrichtung zu messenden) Länge desjenigen Bereichs der Elektrode 20 einnimmt, in dem die Schmalseiten 25, 26 von der Spitze 22 weg divergieren. Die Überzüge 29, 30 können sich in diesen Bereich hinein und vorzugsweise bis zur Spitze 22 erstrecken. Damit wird der Funken bzw. Plasmastrom elektrisch direkt von der Beschichtung 29, 30 gespeist. Die Dicke der Überzüge 29, 30 kann relativ gering sein. Es hat sich gezeigt, dass schon Beschichtungen von 10 bis 20 µm Dicke zu einer wesentlichen Verlängerung der Standzeit der Elektrode 20 und zu einem wesentlich verringerten Materialabtrag und stark verringerter Wärmeabstrahlung von derselben führen. Vorzugsweise beträgt die Dicke der beispielsweise aus Silber bestehenden Überzüge 20 µm, 30 µm oder 50 µm. Der Überzug hat vorzugsweise einen Wärmeleitwert von über 400W/(m*K). Die Dicke der Elektrode kann z.B. 0,1 mm betragen. Auch der Wärmeleitwert der gesamten Elektrode überschreitet vorzugsweise 400W/(m*K). Die aus der Materialkombination Edelstahl/Silber bestehende Elektrode 20 weist dadurch eine überragende Standzeit auf.

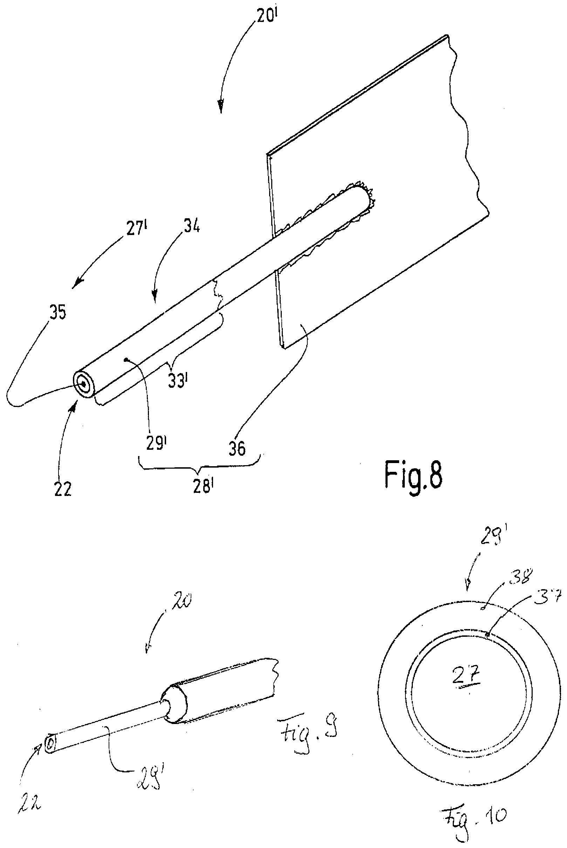

Es ist bei einer abgewandelten nicht beansrpuchten Ausführungsform auch möglich, den Elektrodenquerschnitt, anders als bei den vorstehend beschriebenen Ausführungsformen, in proximaler Richtung nicht kontinuierlich, sondern sprunghaft, d.h. in einer oder in mehreren Stufen zunehmen zu lassen. Eine solche Ausführungsform ist in Figur 8 veranschaulicht. Jedoch verwirklicht auch diese Ausführungsform das erfindungsgemäße Konzept, weswegen bei der Beschreibung dieser Elektrode 20' auf die Beschreibung der Ausführungsform nach Figur 1 bis 7 verwiesen wird. Die bereits eingeführten Bezugszeichen werden weitergeführt, wobei diese zur Unterscheidung jeweils mit einem Apostroph versehen sind. Die vorstehende Beschreibung gilt mit Ausnahme der nachfolgenden Besonderheiten entsprechend für die Ausführungsform nach Figur 8.

Die Elektrode 20' weist eine Spitze 22' auf, die hier durch das spitze oder stumpfe Ende eines drahtförmigen, geraden oder gewellten Elektrodenabschnitts gebildet sein kann. Dieser drahtförmige Elektrodenabschnitt 34 enthält eine Seele 35, die den Grundkörper 27' bildet und ihrerseits als dünner Zylinderstift ausgebildet sein kann. Der Durchmesser des drahtförmigen Elektrodenabschnitts ist vorzugsweise geringer als 0,5 mm und beträgt z.B. 0,3 mm. Die Seele 35 ist mit einem Überzug 29' versehen, der hier, gegebenenfalls in Verbindung mit einem Elektrodenhalteplättchen 36, die Wärmeableiteinrichtung 28' bildet. Der Elektrodenabschnitt 34 kann mit dem Elektrodenhalteplättchen 36 verschweißt, verkrimpt oder anderweitig verbunden sein. Eine stoffschlüssige Verbindung wird wegen der besseren Wärmeübergabe bevorzugt. Der Elektrodenhalteabschnitt 36 kann aus Edelstahl oder einem anderen Material bestehen, das mit einer wärmeleitfähigen Beschichtung, wie Wolfram, Kupfer, Aluminium, DLC oder ähnlichem, versehen oder aus wärmeleitfähigem Material, wie Wolfram, Kupfer, Aluminium, DLC oder ähnlichem, ausgebildet ist. An der Übergangsstelle von dem drahtförmigen Elektrodenabschnitt zu dem Elektrodenhalteplättchen 36 nimmt der Querschnitt der Elektrode sprunghaft zu.

Die Elektrode 20 kann auch gemäß Figur 9 und 10 gestaltet sein und in axialem Abstand im Querschnitt kreisförmige Abschnitte mit unterschiedlichen Durchmessern aufweisen.

Bei allen Elektroden 20, 20' gilt unabhängig von der geometrischen Form und unabhängig davon, ob der Elektrodenquerschnitt in proximaler Richtung kontinuierlich oder in Stufen zunimmt oder ob er konstant bleibt oder stellenweise abnimmt, dass der Überzug 29, 29', 30 die Lebensdauer der Elektrode 20, 20' und des Instruments 10 wesentlich erhöht. Dabei ist es insbesondere vorteilhaft, wenn der Überzug 29, 29', 30 sich von der Spitze 22 wenigstens etwa 5 mm bis 10 mm oder auch etwa 10 bis 20 mm in proximaler Richtung erstreckt. Vorzugsweise besteht der Überzug 29, 29', 30 aus einem Metall, z.B. Silber, dessen Schmelztemperatur T_{ü} niedriger ist, als die Schmelztemperatur T_{G} des Grundmaterials, z.B. Edelstahl. Auch weist der Überzug 29, 29', 30 vorzugsweise eine Wärmeleitfähigkeit λ_{ü} auf, die höher ist, als die Wärmeleitfähigkeit λ_{G} des Grundmaterials.

Weiter kann der Überzug 29, 29', 30 aus einer in direktem Kontakt zu dem Grundkörper 27 angeordneten, haftungsbeeinflussenden Zwischenschicht 37 und einer auf der Zwischenschicht 37 angeordneten Oberflächenschicht 38 bestehen. Die Oberflächenschicht 38 besteht vorzugsweise aus einem Metall, dessen Schmelztemperatur T_{O} niedriger als, oder etwa so hoch ist, wie die Schmelztemperatur T_{Z} der Zwischenschicht, die wiederum ihrerseits niedriger ist, als die Schmelztemperatur T_{G} des Materials des Elektroden-Grundkörpers 27. Die Oberflächenschicht 38 besteht außerdem vorzugsweise aus einem Material, dessen Wärmeleitfähigkeit λ_{O} mindestens so hoch ist, wie die Wärmeleitfähigkeit λ_{Z} der Zwischenschicht 37. Die Wärmeleitfähigkeit λ_{Z} der Zwischenschicht 37 ist vorzugsweise höher, als die Wärmeleitfähigkeit λ_{G} des Materials des Elektroden-Grundkörpers 27.

Bei allen vorbeschrieben Elektroden 20, 20' gilt außerdem erfindungsgemäß, dass die Querschnittsfläche A_{Ü} des Überzugs 29, 29' 30 wenigstens an der Spitze 22 (bis etwa 2,5 mm in proximaler Richtung) wenigstens 10 bis 12 % der Querschnittsfläche A_{G} des Elektroden-Grundkörpers aufweist. Außerdem weist die Elektrode 20, 20' einen von ihrer Spitze 22 ausgehenden, sich in proximaler Richtung um wenigstens 2,5 mm erstreckenden Abschnitt auf, dessen Wärmekapazität kleiner als 4,17 mJ/K ist. Die Elektrode 20, 20' weist ein Volumen V_{E} auf, das vorzugsweise in einem bestimmten Verhältnis zu der Größe der Oberfläche A_{ÜO} des Überzugs 29, 29', 30 steht. Vorzugsweise ist das Verhältnis der Oberflächengröße A_{ÜO} zu dem Volumen V_{E} größer als 2,24 mm⁻¹.

Bei Betrieb eines Instruments 10 mit einer Elektrode 20' nach Figur 8, 9 oder 10 geht eine elektrische Entladung und somit der sich ausbildende Funken- oder Plasmastrom zunächst von der Spitze 22 und dann von zumindest einem Teil des drahtförmigen Elektrodenabschnitts 34 aus. Die elektrisch und thermisch leitfähige Beschichtung 29', die vorzugsweise eine Silberbeschichtung ist, setzt den elektrischen Widerstand der Elektrode 20 bzw. 20' spürbar herab. Der hochfrequente Wechselstrom des Generators 13 konzentriert sich in den äußeren Schichten der Elektrode 20, 20' und durchfließt so im Wesentlichen die Beschichtung 29, 30, 29'. Damit werden die ohmschen Verluste an der Elektrode 20, 20' minimiert und zudem wird die reduzierte Wärmemenge durch die Beschichtung wesentlich besser von dem Entladungsfuß weg geleitet und so verteilt, dass sie großflächig auf dem Gasstrom übertragen werden kann. Die Oberflächenschicht 38 und eventuell auch die Zwischenschicht 27 können aufschmelzen und sich etwas von der Spitze 22 in proximaler Richtung zurückziehen. Der Entladungsfußpunkt 33 bleibt an der Spitze 22 (und dem sich daran unmittelbar anschließenden Bereich, ungefähr 2,5 mm) stationär. Dies vermindert die thermische Belastung sowohl der Elektrode 20, 20' als auch des Instruments 10.

Bei einem verbesserten Instrument 10 ist die Elektrode 20, 20' mit einer Wärmeableiteinrichtung 28, 28' in Gestalt eines ein- oder mehrlagigen Überzugs 29, 29', 30 versehen. Dieser weist gegenüber dem Material des Elektrodengrundkörpers 27, 27' vorzugsweise eine höhere elektrische Leitfähigkeit wie auch eine höhere thermische Leitfähigkeit weist. Außerdem weist er vorzugsweise eine niedrigere Schmelztemperatur als das Material des Elektroden-Grundkörpers 27 auf. Die Schmelztemperatur T_{Ü} des Überzugs liegt vorzugsweise unter 1100°C. Ist der Überzug 29, 29', 30 mehrlagig, liegt die Schmelztemperatur T_{O} der äußeren Oberflächenschicht 38 vorzugsweise ebenfalls unter 1100°C, weiter vorzugsweise unter 1000°C.

### Bezugszeichen:

- 10: Instrument
- 11: Gerät
- 12: Gasquelle
- 13: Generator
- 14: Leitung (für Strom)
- 15: Leitung (für Gas)
- 16: Neutralelektrode
- 17: distales Ende des Instruments 10
- 18: Schlauch
- 19: Lumen
- 20: Elektrode
- 21: Draht
- 22: Spitze
- q: Querabmessung der Elektrode 20
- d: Dicke der Elektrode 20
- 23, 24: Flachseiten der Elektrode 20
- 25, 26: Schmalseiten der Elektrode 20
- Q: Querschnitt der Elektrode 20
- α: Winkel zw. Teilen der Schmalseiten 25, 26
- D: Distale Richtung
- P: Proximale Richtung
- λ: Wärmeleitfähigkeit
- λ_{Ü}: Wärmeleitfähigkeit des Überzugs 29, 29', 30
- λ_{Z}: Wärmeleitfähigkeit der Zwischenschicht 37
- λ_{G}: Wärmeleitfähigkeit des Grundkörpers 27
- λ_{O}: Wärmeleitfähigkeit der Oberflächenschicht 38
- 27: Elektroden-Grundkörper
- 28: Wärmeableiteinrichtung
- 29, 29' 30: Überzüge
- 31: Gasstrom
- 32: Plasma
- 33: Fußpunkt
- 34: drahtförmiger Elektrodenabschnitt
- 35: Seele
- 36: Elektrodenhalteabschnitt
- 37: Zwischenschicht
- 38: Oberflächenschicht
- T_{Ü}: Schmelztemperatur des Überzugs 29, 29', 30
- T_{G}: Schmelztemperatur des Materials des Elektroden-Grundkörpers 27
- T_{O}: Schmelztemperatur der Oberflächenschicht 38
- T_{Z}: Schmelztemperatur der Zwischenschicht 37

## Patentansprüche

1. Elektrode (20, 20') für ein elektrochirurgisches Instrument (10) zur Plasmakoagulation,
wobei die Elektrode (20, 20'), die eine distal orientierte Spitze (22) aufweist, von der ausgehend der Elektrodenquerschnitt (Q) in proximaler Richtung (P) kontinuierlich oder in wenigstens einer Stufe zunehmend ausgebildet ist,
wobei die Elektrode (20, 20') aus einer Materialkombination besteht, die eine Wärmeleitfähigkeit (λ) aufweist, die größer ist als 20 W/(m*K), und
und wobei die Elektrode (20, 20') aus einem Elektroden-Grundkörper (27) mit einem wärmeleitenden Überzug (29, 29', 30) besteht,
**dadurch gekennzeichnet, dass** die Elektrode als Plättchen ausgeführt ist, und dass der Überzug (29, 29', 30) eine Querschnittsfläche A_{Ü} aufweist und dass das Material des Elektroden-Grundkörpers (27) eine Querschnittsfläche A_{G} aufweist, wobei das Verhältnis der Querschnittsfläche A_{Ü} zu der Querschnittsfläche A_{G} größer als 0,12 ist.

2. Elektrode nach Anspruch 1, wobei die Elektrode (20, 20') eine maximale Querabmessung (q) und an ihrer Spitze (22) einen Rundungsradius (R) aufweist, der kleiner als ein Zehntel der maximalen Querabmessung (q).

3. Elektrode nach einem der vorstehenden Ansprüche, wobei das Plättchen zwei Flachseiten (23, 24) aufweist, die durch Schmalseiten (25, 26) miteinander verbunden sind.

4. Elektrode nach Anspruch 3, wobei die Elektrode (20, 20') mindestens eine Flachseite (23, 24) aufweist und dass der wärmeleitende Überzug (29, 29', 30) durch eine Schicht gebildet ist, die die gesamte Flachseite (23, 24) einnehmend ausgebildet ist.

5. Elektrode nach einem der vorstehenden Ansprüche, wobei der Überzug (29, 29', 30) aus einem wärmeleitfähigen Material, insbesondere einem Metall oder einer Metall-Legierung bestehend ausgebildet ist und dass der Überzug (29, 29', 30) eine thermische und/oder elektrische Leitfähigkeit aufweist, die jeweils größer ist als die thermische und/oder elektrische Leitfähigkeit des Grundkörpers (27, 27').

6. Elektrode nach einem der vorstehenden Ansprüche, wobei der Überzug (29, 29', 30) sich bis in einen Bereich (33) erstreckend ausgebildet und angeordnet ist, der zum direkten Kontakt mit einem von der Elektrode (20, 20') ausgehenden Funken vorgesehen ist.

7. Elektrode nach einem der vorstehenden Ansprüche, wobei der Überzug (29, 29', 30) aus einem Metall oder einer Metall-Legierung besteht, deren Schmelztemperatur T_{ü} niedriger ist, als die Schmelztemperatur T_{G} des Materials des Elektroden-Grundkörpers (27).

8. Elektrode nach einem der vorstehenden Ansprüche, wobei der Überzug (29, 29', 30) aus einer in direktem Kontakt zu dem Elektroden-Grundkörper (27) angeordneten Zwischenschicht (27) und einer auf der Zwischenschicht (27) angeordneten Oberflächenschicht (38) besteht.

9. Elektrode nach Anspruch 8, wobei die Oberflächenschicht (38) aus einem Metall oder einer Metall-Legierung besteht, deren Schmelztemperatur T_{O} niedriger ist, als die Schmelztemperatur T_{Z} der Zwischenschicht (37), die wiederum ihrerseits niedriger ist, als die Schmelztemperatur T_{G} des Materials des Elektroden-Grundkörpers (27).

10. Elektrode nach Anspruch 8 oder 9, wobei die Oberflächenschicht (38) aus einem Metall oder einer Metall-Legierung besteht, deren Wärmeleitfähigkeit λ_{D} höher ist, als die Wärmeleitfähigkeit λ_{Z} der Zwischenschicht (37), die wiederum ihrerseits höher ist, als die Wärmeleitfähigkeit λ_{G} des Materials des Elektroden-Grundkörpers.

11. Elektrode nach einem der vorstehenden Ansprüche, wobei die Elektrode (20, 20') einen von ihrer Spitze (22) ausgehenden, sich in proximaler Richtung um wenigstens 2,5mm erstreckenden Abschnitt aufweist, dessen Wärmekapazität kleiner als 4,17 mJ/K ist.

12. Elektrode nach einem der vorstehenden Ansprüche, wobei die Elektrode (20, 20') ein Volumen V_{E} aufweist und dass der Überzug (29, 29', 30) eine Oberflächengröße A_{ÜO} aufweist, wobei das Verhältnis der Oberflächengröße A_{ÜO} zu dem Volumen V_{E} größer als 2,24 mm⁻¹ ist.

13. Instrument (10) mit einer Elektrode (20, 20') nach einem der vorstehenden Ansprüche.

14. Instrument (10) nach Anspruch 13, wobei es ein Rohr oder Schlauch (18) aufweist, das oder der ein Lumen (19) umgibt, das an dem distalen Ende (17) des Rohrs oder Schlauchs (18) offen ist und das an eine Gasquelle, insbesondere eine Argonquelle anschließbar ist, wobei die Elektrode (20, 20') ganz oder teilweise in dem Lumen (19) angeordnet und mit einem Generator (13) verbindbar ist.

## Claims

1. Electrode (20, 20') for an electrosurgical instrument (10) for plasma coagulation,
wherein the electrode (20, 20') comprises a tip (22) orientated in distal direction, wherein the electrode cross-section (Q) is configured in a manner increasing continuously or in at least one step starting from the tip (22) in proximal direction (P),
wherein the electrode (20, 20') consists of a material combination, the thermal conductivity (λ) of which is larger than 20 W/(m*K) and
wherein the electrode (20, 20') consists of an electrode base body (27) having a thermally conductive overlay (29, 29', 30),
**characterized in that** the electrode (20, 20') is configured as platelet and that the overlay (29, 29', 30) comprises a cross-section area A_{Ü} and that the material of the electrode base body (27) comprises a cross-section area A_{G}, wherein the ratio between the cross-section area A_{Ü} to the cross-section area A_{G} is larger than 0.12

2. Electrode according to claim 1, wherein the electrode (20, 20') has a maximum transverse dimension (q) and a radius of curvature (R) at its tip (22) that is smaller than one tenth of the maximum transverse dimension (q).

3. Electrode according to any of the preceding claims, wherein the platelet comprises two flat sides (23, 24) that are connected with one another by means of narrow sides (25, 26).

4. Electrode according to claim 3, wherein the electrode (20, 20') comprises at least one flat side (23, 24) and that the thermally conductive overlay (29, 29', 30) is formed by a layer that is configured in a manner to cover the entire flat side (23, 24).

5. Electrode according to any of the preceding claims, wherein the overlay (29, 29', 30) is made of a thermally conductive material, particularly consisting of a metal or a metal alloy, and that the overlay (29, 29', 30) comprises a thermal and/or electrical conductivity that is respectively higher than the thermal and/or electrical conductivity than the base body (27, 27').

6. Electrode according to any of the preceding claims, wherein the overlay (29, 29', 30) is configured and arranged to extend up to section (33) that is provided for direct contact with a spark originating from the electrode (20, 20').

7. Electrode according to any of the preceding claims, wherein the overlay (29, 29', 30) consists of a metal or a metal alloy, the melting temperature T_{Ü} of which is lower than the melting temperature T_{G} of the material of the base body (27).

8. Electrode according to any of the preceding claims, wherein the electrode (20, 20') is configured as platelet and that the overlay (29, 29', 30) consists of an intermediate layer (27) that is in direct contact with the electrode base body (27) and a surface layer (38) arranged on the intermediate layer (27).

9. Electrode according to claim 8, wherein the surface layer (38) consists of a metal or a metal alloy, the melting temperature T_{O} of which is lower than the melting temperature T_{Z} of the intermediate layer (37) that in turn is lower than the melting temperature T_{G} of the material of the electrode base body (27).

10. Electrode according to claim 8 or 9, wherein the surface layer (38) consists of a metal or a metal alloy, the thermal conductivity λ_{O} of which is higher than the thermal conductivity λ_{Z} of the intermediate layer (37) that in turn is higher than the thermal conductivity λ_{G} of the material of the electrode base body.

11. Electrode according to any of the preceding claims, wherein the electrode (20, 20') comprises a section extending from its tip (22) in proximal direction along at least 2.5 mm, the thermal capacity of which is lower than 4.17 mJ/K.

12. Electrode according to any of the preceding claims, wherein the electrode (20, 20') comprises a volume V_{E} and that the overlay (29, 29', 30) comprises a surface area A_{ÜO}, wherein the ratio of the surface area A_{ÜO} to the volume V_{E} is larger than 2.24 mm⁻¹.

13. Instrument (10) having an electrode (20, 20') according to any of the preceding claims.

14. Instrument (10) according to claim 13, wherein it comprises a tube or hose (18) that surrounds a lumen (19) that is open at the distal end (17) of the tube or hose (18) and that can be connected to a gas source, particularly an argon source, wherein the electrode (20, 20') is completely or partly arranged in the lumen (19) and can be connected to a generator (13).

## Revendications

1. Électrode (20, 20') pour un instrument électrochirurgical (10) de coagulation de plasma, dans laquelle l'électrode (20, 20') présente une pointe (22) orientée distalement, à partir de laquelle la section transversale d'électrode (Q) est réalisée de manière croissante en continu ou par au moins un palier dans une direction proximale (P),
dans laquelle l'électrode (20, 20') est constituée d'une combinaison de matériaux, qui présente une conductivité thermique (λ), qui est supérieure à 20 W/(m*K), et
et dans laquelle l'électrode (20, 20') est constituée d'un corps de base d'électrode (27) avec un revêtement thermoconducteur (29, 29', 30),
**caractérisée en ce que** l'électrode est réalisée sous forme de plaquette, et que le revêtement (29, 29', 30) présente une surface de section transversale A_{Ü} et que le matériau du corps de base de l'électrode (27) présente une surface de section transversale A_{G}, dans laquelle le rapport de la surface de section transversale A_{Ü} à la surface de section transversale A_{G} est supérieur à 0,12.

2. Électrode selon la revendication 1, dans laquelle l'électrode (20, 20') présente une dimension transversale maximale (q) et, sur sa pointe (22), un rayon d'arrondi (R) qui est inférieur à un dixième de la dimension transversale maximale (q).

3. Électrode selon l'une des revendications précédentes, dans laquelle la plaquette présente deux côtés plats (23, 24), qui sont reliés l'un à l'autre par des côtés étroits (25, 26).

4. Électrode selon la revendication 3, dans laquelle l'électrode (20, 20') présente au moins un côté plat (23, 24) et que le revêtement thermoconducteur (29, 29', 30) est formé par une couche qui est réalisée de manière à occuper la totalité du côté plat (23, 24).

5. Électrode selon l'une quelconque des revendications précédentes, dans laquelle le revêtement (29, 29', 30) est constitué d'un matériau thermoconducteur, en particulier d'un métal ou d'un alliage métallique, et que le revêtement (29, 29', 30) présente une conductivité thermique et/ou électrique qui est respectivement supérieure à la conductivité thermique et/ou électrique du corps de base (27, 27').

6. Électrode selon l'une quelconque des revendications précédentes, dans laquelle le revêtement (29, 29', 30) est réalisé et disposé de manière à s'étendre jusque dans une zone (33), qui est prévue pour le contact direct avec une étincelle émanant de l'électrode (20, 20').

7. Électrode selon l'une quelconque des revendications précédentes, dans laquelle le revêtement (29, 29', 30) est constitué d'un métal ou d'un alliage métallique dont la température de fusion T_{ü} est inférieure à la température de fusion T_{G} du matériau du corps de base d'électrode (27).

8. Électrode selon l'une quelconque des revendications précédentes, dans laquelle le revêtement (29, 29', 30) est constitué d'une couche intermédiaire (27) disposée en contact direct avec le corps de base de l'électrode (27) et d'une couche de surface (38) disposée sur la couche intermédiaire (27).

9. Électrode selon la revendication 8, dans laquelle la couche de surface (38) est constituée d'un métal ou d'un alliage métallique dont la température de fusion To est inférieure à la température de fusion T_{z} de la couche intermédiaire (37), qui est elle-même inférieure à la température de fusion T_{G} du matériau du corps de base de l'électrode (27).

10. Électrode selon la revendication 8 ou 9, dans laquelle la couche de surface (38) est constituée d'un métal ou d'un alliage métallique dont la conductivité thermique λ_{D} est supérieure à la conductivité thermique λ_{z} de la couche intermédiaire (37), qui est elle-même supérieure à la conductivité thermique λ_{G} du matériau du corps de base d'électrode.

11. Électrode selon l'une quelconque des revendications précédentes, dans laquelle l'électrode (20, 20') présente une section partant de sa pointe (22) et s'étendant sur au moins 2,5 mm dans la direction proximale, dont la capacité thermique est inférieure à 4,17 mJ/K.

12. Électrode selon l'une quelconque des revendications précédentes, dans laquelle l'électrode (20, 20') présente un volume V_{E} et le revêtement (29, 29', 30) présente une surface A_{ÜO}, dans laquelle le rapport entre la surface A_{ÜO} et le volume V_{E} est supérieur à 2,24 mm⁻¹.

13. Instrument (10) avec une électrode (20, 20') selon l'une quelconque des revendications précédentes.

14. Instrument (10) selon la revendication 13, dans lequel il présente un tube ou un tuyau flexible (18), qui entoure une lumière (19) qui est ouverte sur l'extrémité distale (17) du tube ou du tuyau flexible (18) et qui peut être raccordée à une source de gaz, en particulier une source d'argon, dans lequel l'électrode (20, 20') est disposée en totalité ou en partie dans la lumière (19) et peut être reliée à un générateur (13).
